(19) Europäisches Patentamt
European Patent Office
Office européen des brevets

(11) Publication number: **0 102 727**
**B1**

(12) **EUROPEAN PATENT SPECIFICATION**

(45) Date of publication of patent specification: **20.01.88**

(51) Int. Cl.$^4$: **C 07 D 401/06,** A 61 K 31/44

(21) Application number: **83304229.4**

(22) Date of filing: **21.07.83**

(54) **Chloropyridyl antifungal agents.**

(30) Priority: **06.11.82 GB 8231751**
**07.08.82 GB 8222849**

(43) Date of publication of application:
**14.03.84 Bulletin 84/11**

(45) Publication of the grant of the patent:
**20.01.88 Bulletin 88/03**

(84) Designated Contracting States:
**BE DE FR GB IT LU NL**

(56) References cited:
**EP-A-0 046 337**
**EP-A-0 060 223**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE DE FR IT LU NL**

(72) Inventor: **Richardson, Kenneth, Dr.**
**48 St Stephens Hill**
**Canterbury Kent (GB)**
Inventor: **Whittle, Peter John, Dr.**
**5 Winchester Gardens**
**Canterbury Kent (GB)**

(74) Representative: **Moore, James William, Dr.**
**Pfizer Limited Ramsgate Road**
**Sandwich Kent CT13 9NJ (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 102 727 B1

**Description**

This invention relates to chloropyridyl antifungal agents and in particular to certain 1-(5-chloropyrid-2-yl)-1-(2,4-dihalophenyl)-2-(1,2,4-triazol-1-yl) ethanol compounds which are particularly effective as oral and topical agents in the treatment of fungal diseases in humans and other animals, and to pharmaceutical compositions containing such compounds.

European Patent Application no. 0046337 discloses a broad class of triazole compounds having the general formula:

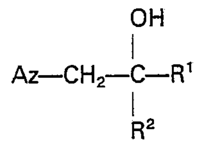

formula (I)

or stereoisomers thereof, wherein $R^1$ is alkyl, cyloalkyl or optionally substituted phenyl; $R^2$ is a heterocyclic group; and Az is a 1,2,4-triazolyl radical; and acid addition salts, ethers, esters and metal complexes thereof. Az represents a 1,2,4-triazol-l-yl radical or a 1,2,4-triazol-4-yl radical.

These compounds are stated to possess fungicidal activity as plant and pharmaceutical fungicides, and also to be plant growth regulators. They are also stated to possess antifungal properties which are useful in the treatment of candidiasis (e.g. caused by *Candida albicans*) and human dermatophyte infections.

It has now been found that two specific unexemplified compounds within this broad class have higher *in vivo* activity against Candida albicans than the structurally closest related compounds illustrated in the Examples of European Patent Application no. 0046337. This surprising degree of activity makes the compounds of particular value in the systemic treatment of human fungal infections. To reach these compounds it is necessary to select specific values for $R^1$ and $R^2$ from the wide range of values specified, and in particular it is necessary to select for $R^2$ a 5-chloropryid-2-yl group, a particular substituent group which is not disclosed in the European Patent Application referred to.

The chloropyridyl compounds of the present invention have the formula (II).

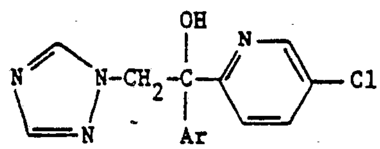

Formula (II)

where Ar is 2,4-dichlorophenyl or 2,4-difluorophenyl, and pharmaceutically acceptable salts thereof.

The compound wherein Ar is 2,4-difluorophenyl is preferred.

The invention also provides pharmaceutical compositions, especially compositions for oral administration, comprising a compound of the formula (II) or a pharmaceutically acceptable salt thereof, together with a pharmaceutically acceptable diluent or carrier.

The compounds of formula (II) can be prepared according to the following reaction scheme wherein Ar is as previously defined:

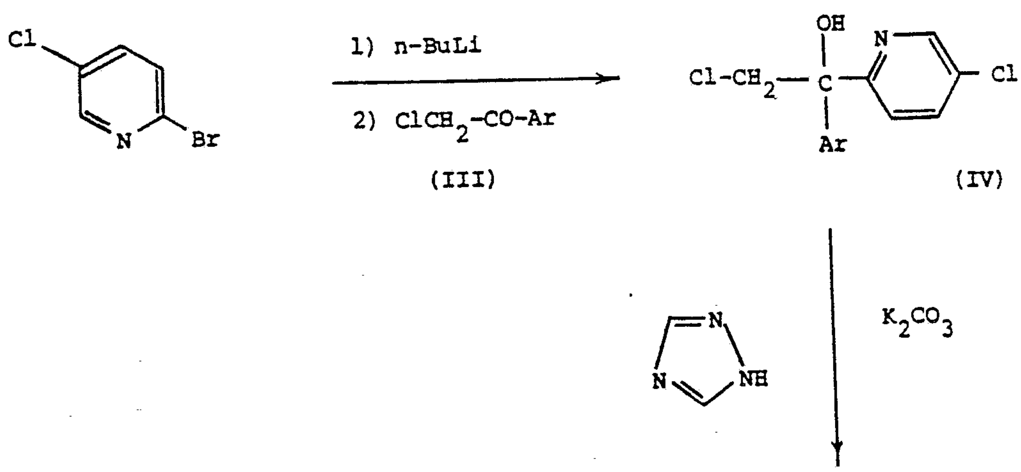


(II)

The reaction is simply performed by first reacting 2-bromo-5-chloropyridine with n-butyl lithium in an inert organic solvent, e.g. diethylether, at a low temperature, e.g. −78°C, and then adding the appropriate 2-chloro-2′,4′-dihaloacetophenone (III). The intermediate (IV) is generally not isolated in pure form but is reacted directly with triazole and anhydrous potassium carbonate in dimethylformamide. The reaction is assisted by warming, e.g. at 80°C, and after several hours the desired product may be isolated in a conventional manner and further purified, if desired, by crystallization or by chromatography.

The compounds of the invention contain a chiral centre, and the invention includes both resolved and unresolved forms.

Pharmaceutically acceptable salts of the compounds of the formula (II) include those formed from strong acids which form non-toxic acid addition salts, such as hydrochloric, hydrobromic, sulphuric, nitric, oxalic and methanesulphonic acids.

The salts may be obtained by conventional procedures, e.g. by mixing solutions containing equimolar amounts of the free base and desired acid, and the required salt is collected by filtration, if insoluble, or by evaporation of the solvent.

The compounds of the formula (II) and their pharmaceutically acceptable salts are anti-fungal agents, useful in combating fungal infections in animals, including humans. For example they are useful in treating topical fungal infections in man caused by, among other organisms, species of *Candida, Trichophyton, Microsporum,* or *Epidermophyton,* or in mucosal infections caused by *Candida albicans* (e.g. thrush and vaginal candidiasis). They may also be used systemically in the treatment of systemic fungal infections caused by for example, *Candida albicans, Cryptococcus neoformans, Aspergillus fumigatus, Coccidioides, Paracoccidioides, Histoplasma* or *Blastomyces.*

The *in vitro* evaluation of the anti-fungal activity of the compounds can be performed by determining the minimum inhibitory concentration (m.i.c.) of the test compounds in a suitable medium at which growth of the particular micro-organism fails to occur. In practice, a series of agar plates, each having the test compound incorporated at a particular concentration are inoculated with a standard culture of, for example, *Candida albicans* and each plate is then incubated for 48 hours at 37°C. The plates are then examined for the presence or absence of growth of the fungus and the appropriate m.i.c. value is noted. Other micro-organisms used in such tests can include *Cryptococcus neoformans, Aspergillus fumigatus, Trichophyton* spp; *Microsporum* spp; *Epidermophyton floccosum, Coccidioides immitis,* and *Torulopsis glabrata.*

The *in vivo* evaluation of the compounds can be carried out at a series of dose levels by intraperitoneal or intravenous injection or by oral administration, to mice which are inoculated with a strain of *Candida albicans.* Activity is based on the survival of a treated group of mice after the death of an untreated group of mice following 48 hours observation. The dose level at which the compound provides 50% protection against the lethal effect of the infection is noted.

Results obtained for the compounds of formula (II) in such tests show that they are more effective as oral antifungal agents than structurally closely related compounds disclosed in the Examples of European Patent Application no. 0046337, as can be seen from the following table which gives comparative results for 2 of the compounds of European Patent Application No. 0046337, together with the results for Examples 1 and 2 of the present application when tested orally in mice infected with *Candida albicans.*

| Compound | Structure | $PD_{50}$ mg/kg (oral administration) |
|---|---|---|
| A | Formula I<br>$R^1$ = 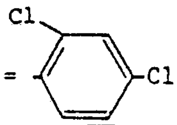<br>$R^2$ = 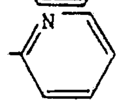 | 1.5 |
| B | Formula I<br>$R^1$ = 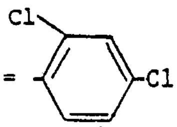<br>$R^2$ = 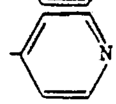 | > 5.0 |
| Example 1 | Formula II<br>Ar = 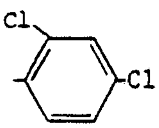 | 0.5 |
| Example 2 | Formula II<br>Ar = 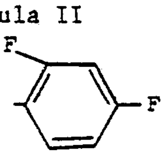 | 0.1 |

For human use, the anti-fungal compounds of the invention can be administered alone, but will generally be administered in admixture with a pharmaceutical carrier selected with regard to the intended route of administration and standard pharmaceutical practice. For example, they may be administered orally in the form of tablets containing such excipients as starch or lactose, or in capsules or ovules either alone or in admixture with excipients, or in the form of a syrup or suspension containing flavouring or colouring agents. They may be injected parenterally, for example, intravenously, intramuscularly or subcutaneously. For parenteral administration, they are best used in the form of a sterile aqueous solution which may contain other substances, for example, enough salts or glucose to make the solution isotonic with blood.

For oral and parenteral administration to human patients, it is expected that the daily dosage level of the antifungal compounds of the invention will be from 0.1 to 10 mg/kg (in divided doses) when administered by either the oral or parenteral route. Thus tablets or capsules of the compounds can be expected to contain from 5 mg to 0.5 g of active compound for administration singly or two or more at a time as appropriate. In any event the physician will determine the actual dosage which will be most suitable for an individual patient and it will vary with the age, weight and response of the particular patient. The above dosages are exemplary of the average case but there can, of course, be individual instances where higher or lower dosage ranges are merited, and such are within the scope of this invention.

Alternatively, the anti-fungal compounds of formula (II) may be administered in the form of a suppository or pessary, or they may be applied topically in the form of a lotion, solution, cream, ointment or dusting powder. For example, they may be incorporated into a cream consisting of an aqueous emulsion

of polyethylene glycols or liquid paraffin; or they may be incorporated, at a concentration between 1 and 10%, into an ointment consisting of a white wax or white soft paraffin base together with such stabilizers and preservatives as may be required.

The following Examples illustrate preparation of the compounds of the invention.

Example 1

1-(2,4-Dichlorophenyl)-1-(5-chloropyrid-2-yl)-2-(1,2,4-triazol-1-yl)ethanol

2-Bromo-5-chloropyridine (6 g, 0.031 M) was stirred in a mixture of diethylether (40 ml) and n-hexane (40 ml) at −78°C. n-Butyllithium (19.2 ml of 1.55 M solution in hexane, 0.03 M) was then added dropwise over 45 minutes and the mixture was stirred for a further 20 minutes at −78°C. A solution of 2-chloro-2′,4′-dichloroacetophenone (5.6 g, 0.025 M) in diethylether (100 ml) was then added dropwise over 45 minutes and the mixture was stirred for a further 1 hour at −78°C before being allowed to warm to 0°C over 2 hours. Glacial acetic acid (6 ml) was added, followed by water (40 ml). The organic layer was separated and the aqueous layer was extracted with diethylether (2 × 40 ml). The combined diethylether fractions were dried over magnesium sulphate and evaporated to give an oil which was dissolved in dimethylformamide (100 ml) containing 1,2,4-triazole (10 g, 0.145 M) and anhydrous potassium carbonate (30 g, 0.217 M). This mixture was stirred and heated at 85°C for 18 hours. The solvent was then removed and the residue was treated with a mixture of ethyl acetate (250 ml) and water (50 ml). The organic layer was separated and the aqueous phase was extracted with ethyl acetate (2 × 50 ml). The combined organic fractions were dried over magnesium sulphate and evaporated to give an oil which was chromatographed on silica eluting with ethyl acetate to give, after one recrystallisation from cyclohexane, the *title compound,* (1.5 g, 16%), m.p. 149—150°C. (found: C,48.43; H,2.95; N,15.06. $C_{15}H_{11}Cl_3N_4O$ requires: C,48.74; H,3.00; N,15.16%).

Example 2

1-(5-Chloropyrid-2-yl)-1-(2,4-difluorophenyl)-2-(1,2,4-triazol-1-yl)ethanol

This compound was prepared in an identical manner to that described in Example 1 but starting with 2-chloro-2′,4′-difluoroacetophenone instead of 2-chloro-2′,4′-dichloroacetophenone to give, after one recrystallisation from hexane, the *title compound* as white crystals (1.35 g, 16%), m.p. 128—130°C. (found: C,53.16; H,3.31; N,16.37. $C_{15}H_{11}ClF_2N_4O$ requires: C,53.56; H,3.29; N,16.64%).

**Claims**

1. A compound of the formula:

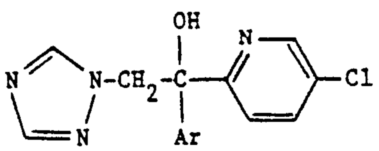

--- (II)

wherein Ar is 2,4-dichlorophenyl or 2,4-difluorophenyl, and pharmaceutically acceptable salts thereof.

2. 1-(5-Chloropyrid-2-yl)-1-(2,4-difluorophenyl)-2-(1,2,4-triazol-l-yl) ethanol.

3. A process for preparing a compound of the formula (II) as defined in claim 1 which comprises reacting a compound of the formula:

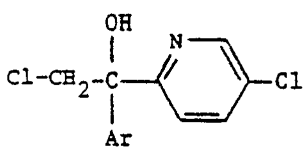

--- (IV)

wherein Ar is defined as in claim 1, with triazole.

4. A pharmaceutical composition comprising a compound of the formula (II) or a pharmaceutically acceptable salt thereof together with a pharmaceutically acceptable diluent or carrier.

5. A pharmaceutical composition for oral administration to a human being in the form of a tablet, capsule, ovule, syrup or suspension containing a compound of the formula (II) as claimed in claim 1 or claim 2, or a pharmaceutically acceptable salt thereof.

6. A compound of the formula (II) as claimed in claim 1 or claim 2 or a pharmaceutically acceptable salt thereof for use in treating a fungal infection in a human being.

**Patentansprüche**

1. Verbindung der Formel

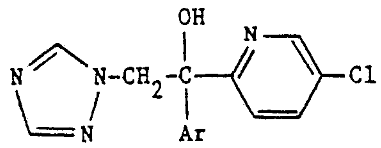

--- (II)

worin Ar 2,4-Dichlorphenyl oder 2,4-Difluorphenyl ist, und pharmazeutisch annehmbare Salze davon.

2. 1-(5-Chlorpyrid-2-yl)-1-(2,4-difluorphenyl)-2-(1,2,4-triazol-1-yl)-ethanol.

3. Verfahren zur Herstellung einer Verbindung der Formel II wie definiert in Anspruch 1, wobei eine Verbindung der Formel

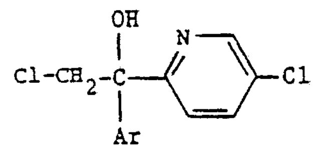

--- (IV)

worin Ar die in Anspruch 1 bezeichnete Definition besitzt, mit Triazol umgesetzt wird.

4. Pharmazeutische Zusammensetzung umfassend eine Verbindung der Formel II oder ein pharmazeutisch annehmbares Salz davon zusammen mit einem pharmazeutisch annehmbaren Verdünnungsmittel oder Träger.

5. Pharmazeutische Zusammensetzung für orale Verabreichung an einen Menschen in Form einer Tablette, Kapsel, Ovulums, Sirups oder Suspension enthaltend eine Verbindung der Formel II gemäß Anspruch 1 oder 2 oder ein pharmazeutisch annehmbares Salz davon.

6. Verbindung der Formel II gemäß Anspruch 1 oder 2 oder ein pharmazeutisch annehmbares Salz davon zur Verwendung bei der Behandlung von Pilzinfektionen in einem Menschen.

**Revendications**

1. Composé de formule:

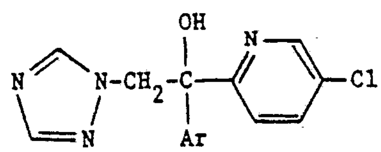

--- (II)

dans laquelle Ar est un groupe 2,4-dichlorophényle ou 2,4-difluorophényle, et ses sels pharmaceutiquement acceptables.

2. 1-(5-chloropyrid-2-yl)-1-(2,4-difluorophényl)-2-(1,2,4-triazol-1-yl)éthanol.

3. Procédé de préparation d'un composé de formule (II) selon la revendication 1, caractérisé en ce qu'il consiste à faire réagir un composé de formule:

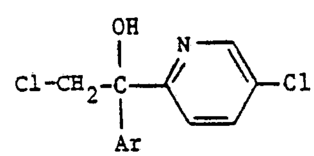

--- (IV)

dans laquelle Ar est tel que défini dans la revendication 1, avec du triazole.

4. Composition pharmaceutique comprenant un composé de formule (II) ou un sel pharmaceutiquement acceptable d'un tel composé, avec un diluant ou un véhicule pharmaceutiquement acceptable.

5. Composition pharmaceutique pour l'administration orale à l'être humain sous la forme de comprimé, gélule, ovule, sirop ou suspension, contenant un composé de formule (II) selon la revendication 1 ou la revendication 2, ou un sel pharmaceutiquement acceptable d'un tel composé.

6. Composé de formule (II) selon la revendication 1 ou 2, ou sel pharmaceutiquement acceptable d'un tel composé, en vue de son application dans le traitement d'une infection fongique chez l'être humain.